# EUROPÄISCHE PATENTSCHRIFT

(11) **EP 3 154 456 B1**
(45) Veröffentlichungstag und Bekanntmachung des Hinweises auf die Patenterteilung: **30.12.2020**
(21) Anmeldenummer: 15741501.9
(22) Anmeldetag: 20.05.2015
(51) Int. Cl.: A61B 17/3205, A61B 17/32, A61B 18/14, A61B 17/22, A61B 18/00

(54) **PAPILLOTOM FÜR DIE PERKUTANE ENDOSKOPISCHE GASTROSTOMIE**
PAPILLOTOME FOR PERCUTANEOUS ENDOSCOPIC GASTROSTOMY
PAPILLOTOME POUR GASTROSTOMIE ENDOSCOPIQUE PERCUTANÉE

(30) Priorität: 10.06.2014 DE 102014211048; 09.09.2014 DE 102014112985
(43) Veröffentlichungstag der Anmeldung: 19.04.2017
(60) Teilanmeldung: 19194912.2 / 3 603 547
(73) Patentinhaber: Kliniken der Stadt Köln gGmbH, 51067 Cologne (DE)
(72) Erfinder: DORMANN, Arno, 51069 Köln (DE)
(74) Vertreter: Patentanwälte Bauer Vorberg Kayser
(86) Internationale Anmeldenummer: PCT/EP2015/061150
(87) Internationale Veröffentlichungsnummer: WO 2015/189017

(56) Entgegenhaltungen:
- DE-A1-102011 085 721
- US-A- 5 163 938
- US-A- 5 396 902
- US-A- 6 017 340
- US-A1- 2008 091 196

## Beschreibung

Die Erfindung betrifft ein Papillotom für die perkutane endoskopische Gastrostomie (PEG) nach dem Oberbegriff des Patentanspruchs 1. Ein derartiges Papillotom ist aus US 6 017 340 A bekannt.

Aus DE 36 43 362 A1, DE 26 57 256 A1, DE 94 09 072 U1 und US6,017,340 A sind Papillotome bekannt. Sie werden endoskopisch in den Körper eines Patienten eingeführt. Insbesondere sind sie zur Entfernung von Gallensteinen vorgesehen. Sie werden auch als Schlingen-Papillotome bezeichnet.

Bei den vorbekannten Papillotomen befindet sich eine vordere Öffnung am freien Ende des Katheters. Der Schneidedraht tritt an diesem freien Ende aus und befindet sich bis zur zweiten Öffnung an der Außenseite des Katheters. Durch Betätigen des Handgriffs wird der Schneidedraht gegenüber dem Katheter verschoben und der Katheter zwischen der vorderen und einer zweiten, hinteren Öffnung gebeugt bzw. U-förmig verformt. Der Schneidedraht verbindet die vordere Öffnung mit der zweiten Öffnung direkt. Zwischen Schneidedraht und dem gebeugten freien Endbereich des Katheters wird eine Schlinge ausgebildet. Der Schneidedraht ist mit einer Hochfrequenz (HF)-Spannungsquelle verbunden. In bekannter Weise und nach dem Stand der Technik kann mit dem Schneidedraht geschnitten werden. Der Schnittweg wird durch die Bewegung des Katheters bestimmt.

Schlingen-Papillotome dieser Art haben sich speziell für die endoskopische Anwendung, insbesondere zur Entfernung von Gallensteinen, bewährt. Dabei wird in der Regel der papilla vaterie bzw. der musculus sphincter oddi durchtrennt. Gewebe, das über ein gewisses Niveau herausschaut, kann durchtrennt werden.

Nach einem Burried Bumper-Syndrom handelt es sich um eine eingewachsene PEG-Halteplatte einer PEG-Sonde. Bei einer PEG-Sonde wird dem Patienten eine Sonde durch die Bauchdecke und die Magenwand (perkutan) in den Magen eingesetzt. Über diese Sonde wird der Patient mit Nahrung und Flüssigkeit versorgt. Die PEG-Sonde weist einen PEG-Katheter auf. Dieser wird außerhalb des Körpers mit einem Anschlusssystem der PEG-Sonde verbunden, das dann über den PEG-Katheter mit dem Mageninneren verbunden ist. Der PEG-Katheter verläuft durch die Bauchdecke und die Magenwand. Bei unzureichender oder falscher Pflege kann es dazu kommen, dass die PEG-Halteplatte von der Magenwand umschlossen und überwachsen wird. Die wird als Burried Bumper Syndrom bezeichnet.

Nach dem Stand der Technik werden eingewachsene PEG-Halteplatten per Laparotomie, mittels des Pull-Verfahrens oder des Push-Verfahrens entfernt.

Unter einer Laparotomie versteht man die chirurgische Öffnung der Bauchdecke. Im Zuge dieser Operation wird auch die Magenwand geöffnet und die eingewachsene PEG-Halteplatte von außen freigeschnitten. Dies ist eine aufwändige, stark invasive und daher risikoreiche Operation, die nur noch in seltenen Fällen angewandt wird.

Das Pull-Verfahren basiert darauf, die eingewachsene PEG-Halteplatte, nach vorherigem Einschneiden des umgebenden Gewebes, durch Zug von außen aus dem Gewebe zu befreien. Durch den liegenden PEG-Katheter wird eine Biopsie Zange zur Versteifung desselben eingeführt und als Widerstand geöffnet. Durch Zug auf die Biopsie Zange wölbt sich die Bauchdecke an der Stelle der eingewachsenen PEG-Halteplatte. Mit einem Skalpell wird die Bauchdecke und ein Teil der Magenwand in der direkten Umgebung des PEG-Katheters bis auf die PEG-Halteplatte durchtrennt. Anschließend wird die PEG-Halteplatte durch Zug von außen, mittels der Biopsie Zange nach außen freigezogen. Dieses Verfahren ist weniger invasiv als eine Laparotomie, jedoch wird auch hierbei noch ein Teil der Bauchdecke und der Magenwand durchtrennt, dies ist mit den damit verbundenen Risiken behaftet.

Beim Push-Verfahren wird unter endoskopischer Kontrolle die PEG-Halteplatte von innen, also aus dem Mageninneren heraus, frei geschnitten. Dazu wird endoskopisch oder perkutan ein Schneidewerkzeug in das Mageninnere eingeführt und die PEG-Halteplatte frei gelegt. Zumeist werden dafür Nadel-Papillotome verwendet, diese besitzen an ihrer Spitze ein nadelartiges Schneidwerkzeug, mit dem das Gewebe durchtrennt werden kann. Sie werden meist endoskopisch eingeführt. Es können auch Schlingen-Papillotome verwendet werden, diese werden perkutan über den PEG-Katheter eingeführt. Dazu werden sie im entspannten Zustand, nicht gebeugt, durch den PEG-Katheter vorgeschoben. Sobald der Papillotom-Katheter weit genug ins Mageninnere (Magenlumen) vorgeschoben ist, wird der Handgriff betätigt und somit die Papillotom Schlinge gebildet. Durch Zurückziehen des Katheters wird der Schneiddraht auf das die PEG-Halteplatte umgebende Gewebe gezogen, dieses kann nun eingeschnitten werden, bis der Schneiddraht auf die PEG-Halteplatte stößt. Meist erfolgen mehrere dieser radiären Schnitte, bis die PEG-Halteplatte frei gelegt ist. Anschließend wird die PEG-Halteplatte mit einem durch den PEG-Katheter vorgeschobenen handelsüblichen Bougie, oder auch Dilatator, in das Mageninnere hinein gedrückt. Die befreite PEG-Halteplatte kann dann endoskopisch entfernt werden.

Bei Bougies handelt es sich um Körperöffnungen oder Gefäß erweiternde medizinische Geräte zumeist aus Edelstahl oder anderen harten Materialien mit einer konischen Form.

Das Push-Verfahren ist das am wenigsten invasive Verfahren und bietet die geringste Komplikationsrate von den dargestellten Verfahren. Allerdings treten auch hier Probleme und Komplikationen auf. Nachteile der Push Technik sind ein häufig vorkommendes Abrutschen bzw. Verrutschen der Schlinge des Papillotoms beim Zurückziehen auf die PEG-Halteplatte, dadurch werden stärkere Blutungen, größere Infektionsherde und ein höherer Arbeitsaufwand ausgelöst. Weiterhin kann die PEG-Halteplatte seitlich nicht frei gelegt werden. Die vorhandenen Bougies sind nicht für die Anwendung mit einer und für eine PEG-Sonde ausgelegt.

Hiervon ausgehend ist es Aufgabe der Erfindung, eine Vorrichtung zur Verfügung zu stellen, mit welcher der Eingriff zielgerichteter und einfacher durchführbar ist, ein Verrutschens des Papillotoms vermieden wird und die Schnitte gezielter gesetzt werden können.

Diese Aufgabe wird gelöst ein Papillotom mit den Merkmalen des Anspruchs 1.

Aufgrund des Überstandes beginnt der Schneidedraht nicht unmittelbar am freien Ende des freien Endbereichs des Katheters, sondern im Abstand vom freien Ende. Dadurch kann der Überstand als Anschlag dienen. Nach Einführen des freien Endbereichs in das Mageninnere ist der freie Endbereich noch im Wesentlichen geradlinig. Wird nun der Handgriff betätigt und dadurch der freie Endereich gebeugt, schlägt der Überstand an einen seitlichen Rand einer Überwachsung der PEG-Halteplatte an. Dadurch kommt es zu einer bestimmten und in gewissem Maße festen Position des Überstandes. Das Papillotom wirkt in seinem freien Endbereich in Art einer Zange, zwischen dem Überstand und dem Katheter jenseits der hinteren Öffnung erfolgt ein Festklemmen. Der dort befindliche Teil der PEG-Halteplatte wird übergriffen und eingespannt. Dadurch ist der Schneidedraht auch in Nähe der vorderen Öffnung abgestützt und fixiert. Er ist nicht nur durch den Katheter selbst gehalten. Zieht nun ein Operateur den Katheter nach außen und führt einen Schnitt durch, so schneidet nicht nur bevorzugt derjenige Teil der quer verlaufenden Schnittkante, der sich in Nähe der zweiten Öffnung befindet, wo sich der Zug insbesondere auswirkt, sondern auch derjenige Teil des Schneidedrahtes, der sich in Nähe der vorderen Öffnung befindet, weil dort die beschriebene Abstützung stattfindet. Insbesondere schneidet auch die längs verlaufende Schnittkante, die sich außerhalb des Mantels der scheibenförmigen PEG-Halteplatte befindet. Durch weiteren Zug am Schneidedraht kann die Schlinge verkleinert und somit die Wirkung der längs verlaufenden Schnittkante gesteuert werden. Durch Ziehen am Katheter kann die Wirkung der quer verlaufenden Schnittkante beeinflusst werden.

Der Schneidedraht ragt gegenüber dem freien Ende nach außen vor. Dieser Bereich des Schneidedrahtes bildet eine Schneidespitze. Diese erfüllt zudem auch folgenden Zweck: Je nach Stärke der Überwachsung der PEG-Halteplatte kann es vorkommen, dass die innere Öffnung des PEG-Katheters in das Mageninnere überwachsen ist. In diesen Fällen löst und durchschneidet man mit der vorderen Schneidespitze beim Einführen des Katheters die Überwachsung nach. So kann der Weg in das Mageninnere frei geschnitten werden. Erst danach ist der freie Endbereich des Papillotoms im Magenlumen. Weiterhin ermöglicht die Schneidspitze ein Verhaken in der Magenwand nach bzw. während der Beugung des Papillotoms. Auch hierdurch wird ein Verrutschen des freien Endbereichs verhindert. Ein derartiges Verrutschen kann auch beim Zurückziehen des Katheters auftreten. Es wird ein unkontrolliertes Einschneiden des Schneidedrahtes in die Überwachsung verhindert, zumindest erschwert. Es werden nicht gezielte und unsaubere Schnitte, größere Wundbereiche und somit vermeidbare Blutungen und Infektionsherde vermieden.

Die Schneidespitze kann auch durch eine Kugel ausgebildet werden. Die Kugel ist am Katheter befestigt und mit dem Schneidedraht elektrisch verbunden. Der Kugeldurchmesser ist möglichst klein gewählt, er ist etwas größer als der lichte Durchmesser des ersten Lumens. Dadurch bleibt die Kugel außen außerhalb des ersten Lumens.

Die längs verlaufende Schnittkante ist bevorzugt durch den Schneidedraht selbst ausgebildet. Sie kann aber auch durch eine Nadel, wie dies beim Nadel-Papillotom der Fall ist, ausgebildet sein. Es kann auch ein Metallstück in das erste Lumen eingeführt sein, das zumindest einen Teil der quer verlaufenden Schnittkante ausbildet. Es ist durchaus möglich und vorgesehen, dass sich die Schneidspitze in einer gewissen Entfernung von z.B. 1-8 mm vom freien Ende des Katheters befindet, also um dieses Maß aus dem ersten Lumen frei hervorragt.

Der Überstand kann ausschließlich durch einen Bereich des Katheters gebildet werden. Er kann zumindest teilweise durch ein Metallstück gebildet sein, das mit dem Katheter mechanisch verbunden ist. Der Überstand kann eine Mischform sein, dann ist der Überstand teilweise durch einen Bereich des Katheters, teilweise durch einen metallischen Gegenstand bestimmt, der aus dem freien Ende hervorragt.

Die quer verlaufende Schnittkante und die längs verlaufende Schnittkante befinden sich in derselben Orientierung zum Katheter. Hiermit ist folgendes gemeint: Blickt man in Axialrichtung auf den Katheter, so befinden sich die beiden Öffnungen in derselben Winkelposition. Damit befindet sich auch der Schneidedraht beider Schnittkanten in derselben Winkelposition.

Vorzugsweise hat das freie Ende des Katheters eine Abschrägung. Der Winkel der Abschrägung beträgt insbesondere etwa 45°, Abweichungen um± 20° sind möglich. Durch die Abschrägung wird eine Spitze gebildet. Diese Spitze hat bevorzugt dieselbe Orientierung zum Katheter wie die vordere Öffnung und die hintere Öffnung.

Die Länge des Überstandes entspricht vorzugsweise zumindest der Dicke der verwendeten PEG-Halteplatte. Diese Länge wird der Dicke angepasst. Bei dünnen Halteplatten kann der Überstand kleiner gewählt werden, bei dicken Halteplatten entsprechend größer. Hinzu kommt noch die Dicke der Überwucherung, die aber nicht immer bekannt ist.

Vorzugsweise ist eine dritte Öffnung im Katheter ausgebildet. Sie befindet sich zwischen der vorderen Öffnung und der hinteren Öffnung. Sie ist in unmittelbarer Nähe, insbesondere 1 bis 8 mm, der vorderen Öffnung. Sie ist ebenso orientiert wie die beiden anderen Öffnungen. Der Draht, der die längs verlaufende Schnittkante bildet, kann durch die dritte Öffnung in den Katheter eingeführt oder aus ihm herausgeführt werden. Die längs verlaufende Schnittkante kann nun so ausgebildet sein, dass sie einen Kreuzungspunkt mit der quer verlaufenden Schnittkante ausbildet. Dadurch wird das Schneiden dort, wo die beiden Schnittkanten zusammenlaufen, verbessert. Die vordere Öffnung wird vorzugsweise nur für den Draht benutzt, der die quer verlaufende Schnittkante ausbildet.

Die quer verlaufende Schnittkante kann auch durch ein Seil gebildet werden. Ein Seil hat den Vorteil, dass es im gebeugten Zustand des freien Bereichs im Wesentlichen geradlinig zwischen den beiden Öffnungen verläuft. Bei einem Schneidedraht, der nur ein Filament hat, wird dies nicht immer vollständig erreicht. Es ist auch möglich, die Schnittkante durch einen dünnen, steifen Metallstift auszubilden, der nicht verformbar ist, der z.B. ähnlich einer Nähnadel ausgeführt ist. Seine Länge muss so bemessen sein, dass er bei stärkster, gewollter Beugung des freien Endbereichs nicht länger ist als der Abstand zwischen den beiden Öffnungen. Eine derartige, nicht verformbare Schnittkante hat den Vorteil, dass sie beim Schneidvorgang weniger nachgibt als ein Draht. Sie kann auch durch ein Metallrohr ausgebildet sein, das über den Schneidedraht geschoben ist und die Länge des Metallstifts hat.

Vorzugsweise ist der Handgriff so ausgebildet, dass der Katheter mit einem stationären Teil des Handgriffs verbunden ist, während ein bewegbarer, insbesondere verschiebbarer Teil des Handgriffs mit dem Schneidedraht verbunden ist. Wird dann der verschiebbare Teil nach außen gezogen, kommt die Beugung zustande. Vorzugsweise weist der Handgriff eine Feststellvorrichtung auf. Durch sie kann die Position eines bewegbaren Teils des Handgriffs gegenüber einem stationären Teil des Handgriffs festgestellt werden.

Ein Feststellmechanismus der Feststellvorrichtung verhindert bzw. erschwert eine Beugung oder Streckung des PEG-Schlingen-Papillotoms. Dieser Feststellmechanismus kann stufenlos, aber auch in Stufen ausgebildet sein. Beispielsweise kann es sich um ein oder mehrere Rastnasen mit einer Vertiefung oder eine Rastnase mit mehreren Vertiefungen handeln. Auch mehrere Rastnasen mit mehreren Vertiefungen sind denkbar. Der Feststellmechanismus kann an einen Radius der PEG-Halteplatte angepasst werden. Bei Anwendung des PEG-Papillotoms wird es, wie oben für bereits vorhandene Schlingen-Papillotome ausgeführt, gebeugt um die Schlinge bzw. die quer verlaufende Schnittkante auszubilden. Durch den Feststellmechanismus kann verhindert werden, dass sich das PEG-Schlingen-Papillotom beim Schneiden aufgrund des Gewebewiderstands wieder streckt. Es kann eine mechanische Spannung in der gebeugten Schlinge aufrecht erhalten werden, so dass der Überstand kraftschlüssig anliegt.

Der Katheter besitzt vorzugsweise eine Länge zwischen 40 und 150 cm, bevorzugt von 50 bis 120 cm. Handelsübliche Papillotome sind aufgrund ihrer endoskopischen Einführung und der Auslegung für den Gallengang deutlich länger ausgebildet. Eine geringere Länge ermöglicht ein ungehindertes Arbeiten. In bekannter Weise ist der Katheter z.B. aus Polytetrafluorethylen hergestellt. Er hat einen Durchmesser von 2 bis 4 mm. Vorzugsweise hat er zwei Lumina, das zweite Lumen wird für einen Führungsdraht verwendet, ein solcher ist Stand der Technik bei einem Push-Verfahren.

Durch den weichen Vorlauf mit abgerundeter Spitze wird sichergestellt, dass der PEG-Bougie beim Einführen in das Mageninnere die gegenüberliegende Magenwand nicht verletzt. Ein eventuell vorhandener Längskanal ist so ausgelegt, dass der Führungsdraht eingeführt werden kann, so dass der PEG-Bougie zielgerichtet in den PEG-Katheter und das Mageninnere eingeführt werden kann. Wichtig ist, dass der PEG-Bougie keine Kanten im Längsverlauf aufweist, damit er sich nicht im PEG-Katheter verhaken kann. Der konische Verlauf sichert dennoch ein Ausdrücken der PEG-Halteplatte in das Mageninnere. Die Steigzahl gibt das Verhältnis vom zweiten Durchmesser zum ersten Durchmesser an.

Der PEG-Bougie kann mit dem Koextrusionsverfahren hergestellt werden.

Der Durchmesser der Spitze bzw. des Vorlaufs liegt vorzugsweise zwischen 30 und 90 % des Innendurchmessers des PEG-Katheters.

Der Vorlauf ist bevorzugt zwischen drei und acht cm lang, besonders bevorzugt zwischen vier und sechs cm. Der PEG-Bougie besitzt bevorzugt eine Gesamtlänge zwischen 10 und 24 cm, besonders bevorzugt zwischen 13 und 18 cm.

Die Steigzahl ist bevorzugt so gewählt, dass der Hauptkörper nach einem bis sechs cm, besonders bevorzugt nach zwei bis vier cm, den Innendurchmesser der PEG-Halteplatte aufweist.

Weitere Vorteile und Merkmale der Erfindung ergeben sich aus den noch nicht angesprochenen Unteransprüchen sowie der nun folgenden Beschreibung mehrerer, nicht einschränkend zu verstehender Ausführungsbeispiele der Erfindung. Diese werden unter Bezugnahme auf die Beschreibung im Folgenden näher erläutert. In der Zeichnung zeigen:
- Fig. 1:: eine Draufsicht auf ein erstes Ausführungsbeispiel eines Papillotoms mit einem gebeugten freien Endbereich, die beiden Schnittkanten sind zu erkennen,
- Fig. 2:: ein axiales Schnittbild durch den freien Endbereich des Katheters nach Fig. 1,
- Fig. 3:: ein axiales Schnittbild ähnlich Fig. 1 durch den freien Endbereich des zweiten Ausführungsbeispiels,
- Fig. 4:: einen radialen Schnitt entlang der Schnittlinie IV-IV in Fig. 3,
- Fig. 5:: ein Schnittbild ähnlich Figur 2 für ein drittes Ausführungsbeispiel, nunmehr mit einem starren Metallkörper, der teilweise den Überstand bildet,
- Fig. 6:: ein axiales Schnittbild eines Teilstücks des freien Endbereichs, nunmehr mit einer Kugel am freien Ende eines ersten Lumens, der Rest ist wie in Fig. 5 ausgeführt,
- Fig. 7:: eine prinzipielle Darstellung eines Teilstücks des freien Endbereichs ähnlich Fig. 5, nunmehr mit einem anderen Metallkörper,
- Fig. 8:: eine perspektivische Darstellung einer PEG-Halteplatte,
- Fig. 9:: einen Schnitt durch eine Magenwand mit eingewachsener PEG-Halteplatte und PEG-Katheter, ein Papillotom ist eingeführt und schnittbereit,
- Fig. 10:: eine Seitenansicht einer ersten Ausführung eines Bougie, und
- Fig. 11: eine Seitenansicht einer zweiten Ausführung eines Bougie.

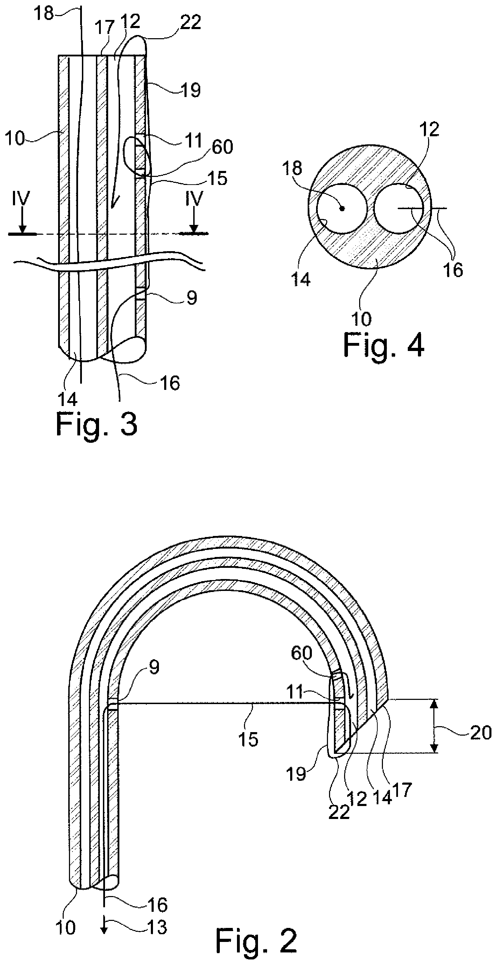

Das Papillotom hat einen Handgriff 2 mit einem stationären Teil 3 und einem bewegbaren Teil 4, das hier als Schieber ausgeführt ist, der am stationären Teil 3 verschiebbar geführt ist. Das stationäre Teil 3 hat eine Öse, am bewegbaren Teil 4 sind zwei entsprechende Öffnungen vorgesehen. In alle diese kann jeweils ein Finger bzw. ein Daumen gesteckt werden, durch Bewegen kann der bewegbare Teil 4 verschoben werden. Am bewegbaren Teil 4 ist ein HF-Anschluss 6 vorgesehen. Die Bewegung ist durch den Pfeil 7 dargestellt. Am unteren Ende des stationären Teils 3 geht dieses über in einen Katheter 10. Er ist zweilumig ausgeführt, siehe Figur 4. Ein zweites Lumen 14 wird über einen Ansatz 8 zugänglich. Dieser schließt mit einem Schraubgewinde ab. Hier kann ein Führungsdraht 18 eingeführt werden.

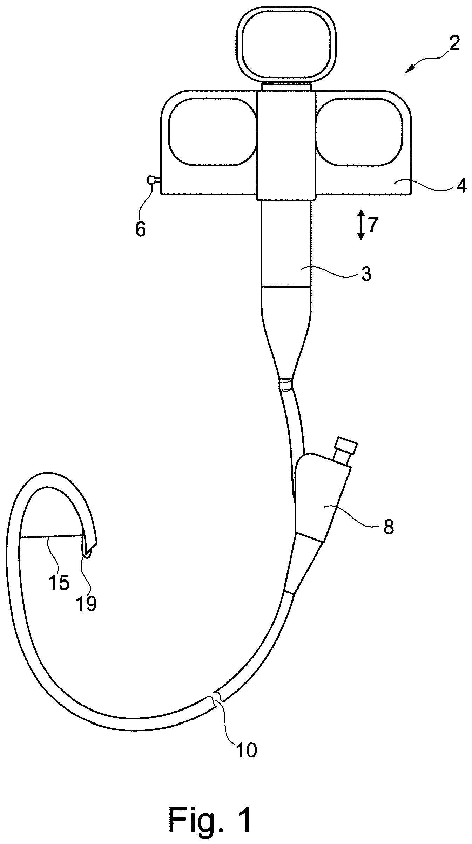

Der Katheter 10 hat einen freien Endbereich. Dieser erstreckt sich von einer vordersten Spitze des Papillotoms bis etwas über eine hintere Öffnung 9 hinaus. Der vordere Endbereich ist der Teil des Katheters 10, der aus einem gestreckten in einen gebeugten Zustand übergeführt werden kann, der gebeugte Zustand ist in Figur 1 gezeigt.

Aus Figur 2 sind Details des freien Endbereichs ersichtlich. In einem ersten Lumen 12 ist ein Schneidedraht 16 verschiebbar geführt. Er ist mit dem bewegbaren Teil 4 und dem HF-Anschluss 6 verbunden. In dem gebeugten Zustand, wie ihn Figur 2 zeigt, wirkt eine Zugkraft entsprechend des Pfeils 13 am Schneidedraht 16. Der Schneidedraht 16 ist zwischen der hinteren Öffnung 9 und einer vorderen Öffnung 11 gespannt, er verläuft dort möglichst gradlinig, er bildet eine quer verlaufene Schnittkante 15 aus. Nach Passieren der vorderen Öffnung 11 verläuft der Schneidedraht 16 im ersten Lumen 12 zu einem freien Ende 17 hin, dort tritt er aus dem ersten Lumen 12 aus und ist um das dortige Schlauchende des Katheters 10 herumgebogen. Er ist an der Außenseite entlang geführt. Er tritt durch eine dritte Öffnung 60 wieder in das erste Lumen 12 ein. Die dritte Öffnung 60 befindet sich zwischen der vorderen Öffnung 11 und der hinteren Öffnung 9. Sie ist in sehr kurzem Abstand von der vorderen Öffnung 11. Der Abstand liegt im Bereich von 1 bis 8 mm. Die dritte Öffnung 60 ist ebenso orientiert zum Katheter 10 wie die beiden anderen Öffnungen 9, 11. Nach Durchtritt durch die dritte Öffnung ist der Schneidedraht 16 tiefer in das erste Lumen 12 hineingesteckt und eventuell durch eine Abwinklung (wie gezeichnet) gesichert.

Im zweiten Ausführungsbeispiel nach den Figuren 3 und 4 ist das freie Ende nicht in einem spitzen Winkel abgeschnitten, wie insbesondere aus Figur 2 ersichtlich ist, vielmehr ist es im Winkel von 90° abgeschnitten. Der Winkel gemäß Figur 2 kann zwischen 10 und 80° liegen, er liegt vorzugsweise bei 45°. In das zweite Lumen 14 ist ein Führungsdraht 18 eingebracht. Der Verlauf des Schneidedrahts 16 ist ähnlich wie im ersten Ausführungsbeispiel, jedoch mit folgender Abweichung: Nach Passieren der vorderen Öffnung 11 verläuft der Schneidedraht 16 im ersten Lumen 12 zur hinteren Öffnung 11 hin und tritt an der dritten Öffnung 60 aus dem ersten Lumen 12 aus und ist um das freie Ende 17 des Katheters 10 herumgebogen und in das erste Lumen 12 hineingesteckt. Darin hat er eine beliebige Länge. Er ist eventuell durch eine Abwinklung gesichert. Diese kann mit dem kurzen Stück zwischen der vorderen Öffnung 11 und der dritten Öffnung 60 des Schneidedrahts 16 oder dem Stück des Schneidedrahts 16 jenseits der hinteren Öffnung 9 verhakt sein.

Mit 20 ist ein Überstand bezeichnet. Die Länge des Überstandes 20 ist im ersten Ausführungsbeispiel praktisch gleich der Länge zwischen einem Kreuzungspunkt der längs verlaufenden Schnittkante 19 mit der querverlaufenden Schnittkante 15 und einer Schneidspitze 22. Genau genommen ist der Überstand 20 geringfügig länger als der Abstand zwischen der vorderen Öffnung 11 und dem freien Ende, weil der Schneidedraht 16 um das Schlauchende des Katheters 10 herumgebogen ist und dort zur Länge des Überstands beiträgt. An seiner Umlenkung an der Spitze des freien Endes 17 bildet der Schneidedraht 16 die Schneidspitze 22.

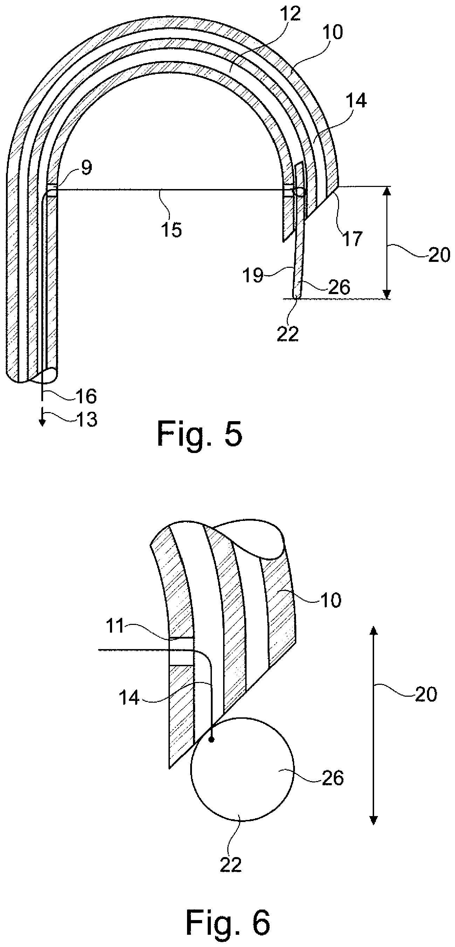

Im dritten Ausführungsbeispiel nach Figur 5 ist der Überstand 22 teilweise durch ein über die vordere Öffnung 11 vorstehendes Teilstück des Katheters 10, teils durch ein Metallstück 26, das im Wesentlichen als Nadel ausgebildet ist, bestimmt. Dieses Metallstück 26 hat einen Außendurchmesser, der etwa dem lichten Durchmesser des ersten Lumens 12 entspricht. Das Metallstück 26 ist nicht biegsam, es hat die Festigkeit beispielsweise einer Nähnadel. Es ist mit dem Schneidedraht 16 elektrisch verbunden. Der Schneidedraht ist um die Nadel 26 herumgelegt und ragt aus der vorderen Öffnung 11 nach außen vor, er verläuft wie in den vorigen Ausführungsbeispielen unter Ausbildung einer quer verlaufenden Schnittkante 15 zur hinteren Öffnung 9. Die Schneidspitze 22 wird nun durch die außenliegende Spitze des Metallstücks 26 gebildet. Die längs verlaufende Schnittkante 19 erstreckt sich nun nicht mehr über die gesamte Länge des Überstandes 22, sondern nur über einen Teil des Überstandes 22, beispielsweise maximal 80%, vorzugsweise maximal 60%.

Im vierten Ausführungsbeispiel nach Figur 6 wird das Metallstück 26 durch eine Kugel gebildet, die die Schneidspitze 22 ausbildet. Sie ist elektrisch mit dem Schneidedraht 16 verbunden. Sie ist auf das Ende des zweiten Lumens 14 aufgelegt und dort befestigt.

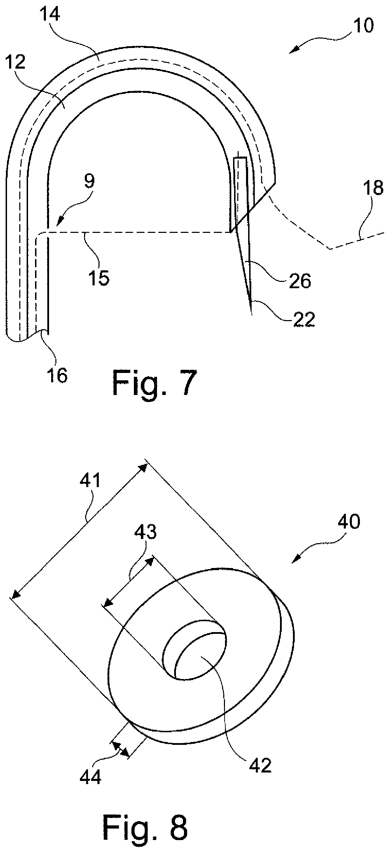

Im fünften Ausführungsbeispiel nach Figur 7 ist das Metallstück 26 als Messer ausgebildet, das selbst schneidet. Man kann wahlweise mit dem Messer mit und ohne am Messer angelegter HF-Spannung schneiden. Im ersten Fall schneiden sowohl die quererlaufende Schnittkante 15 und das Messer, da Schneidedraht 16 und Metallstück 26 elektrisch verbunden sind. Im zweiten Fall schneidet nur das Messer, nun allerdings rein mechanisch. Zudem und unabhängig hiervon bildet das Metallstück 26 allein den Überstand 20. Die vordere Öffnung 11 fällt mit dem freien Ende des ersten Lumens 12 zusammen. Der Schneidedraht 16 ist elektrisch mit dem Metallstück 26 verbunden.

Die Figur 8 zeigt eine handelsübliche PEG-Halteplatte 40. Sie hat einen Durchgang 42. Sie ist als Kreisscheibe ausgebildet. Ihre Dicke wird mit 44 bezeichnet, 41 ist ihr Außendurchmesser, 43 der Lochdurchmesser.

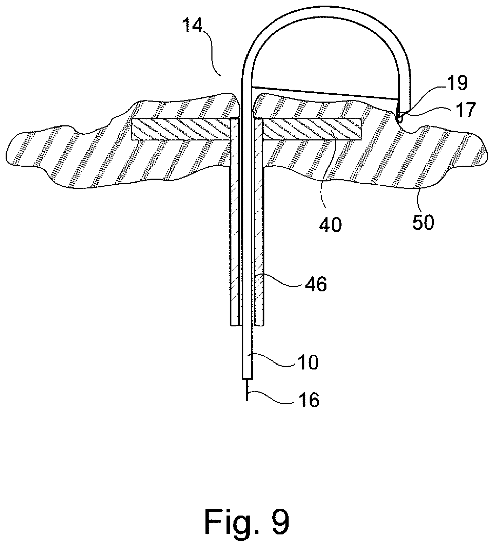

Figur 9 zeigt eine derartige PEG-Halteplatte 40, die in einer Magenwand 50 eingewachsen ist. Die PEG-Halteplatte 40 ist mit einem PEG-Katheter 46 verbunden.

Erkennbar ist, dass der zuvor durch Mukosa überwucherte mageninnere Auslass des PEG-Katheters 46 geöffnet wurde, dies ist mittels der Schneidspitze 22 erfolgt. Das Papillotom ist bereits in einer Position gezeigt, in der ein Schneidvorgang erfolgen kann. Die Schneidspitze 22 ist in Kontakt mit der Muskosa, die quer verlaufende Schnittkante 15 liegt auf der Mukosa innenseitig auf. Das Mageninnere befindet sich in Figur 9 oben. Wird nun HF-Spannung an den Schneidedraht 16 gelegt und der Katheter 10 nach unten gezogen, kommt es zu einem Schneidevorgang. Dieser wird solange ausgeführt, _bis die quer verlaufende Schnittkante 15 in Kontakt mit der PEG-Halteplatte 40 kommt. Dabei kann der Schneidedraht 16 noch weiter in Richtung des Pfeils 13 gezogen werden, um den Schneidvorgang durch die längs verlaufende Schnittkante 19 zu unterstützen. Dies kann fortgesetzt werden, bis es zu einem Kontakt mit dem Umfang der PEG-Halteplatte 40 kommt.

Beide Schnittkanten 15, 19 können somit separat und gezielt eingesetzt werden. Die quer verlaufende Schnittkante 15 wird gesteuert durch eine Bewegung des Katheters 10 in axialer Richtung und ein Drehen des Katheters 10 um die axiale Richtung. Die längs verlaufende Schnittkante 19 wird durch Anziehen in Richtung des Pfeils 13 und Loslassen des Schneidedrahtes 16 in Gegenrichtung hierzu gesteuert.

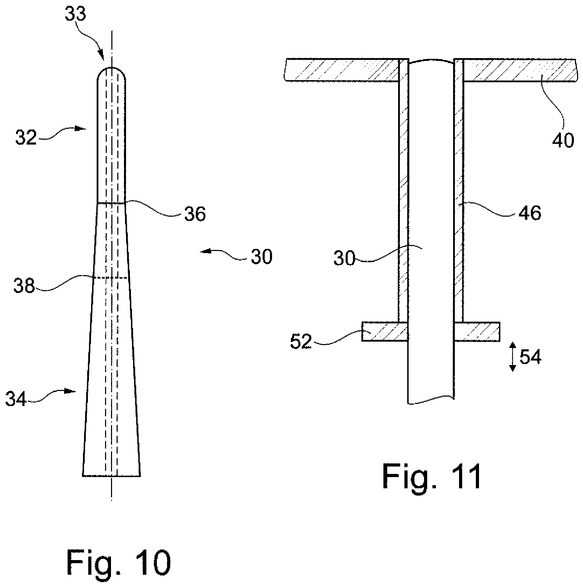

Aus den Figuren 10 und 11 sind zwei Beispiele von sogenannten PEG-Bougies 30 ersichtlich. Diese dienen im Push-Verfahren dazu, die PEG-Halteplatte 40 und den damit verbundenen PEG-Katheter 46 in das Mageninnere vorzuschieben, so dass die PEG-Halteplatte 40 freikommt. Der PEG-Katheter 40 wird dabei soweit wie möglich gekürzt.

Der Bougie mit Figur 10 hat einen Vorlauf 32 und einen Hauptkörper 34, der Vorlauf 32 weist einen konstanten Durchmesser auf. Er hat eine abgerundete Spitze 33. Die Spitze ist aus einem weichen Material hergestellt. Er ist an einem Übergang 36 bündig mit dem Hauptkörper 34 verbunden. Der Hauptkörper 34 hat dort den gleichen Durchmesser wie der Vorlauf 32 und weitet sich konisch vom Übergang 36 nach untenhin aus.

Der PEG-Bougie wird mit der Spitze 33 voran in den PEG-Katheter 46 eingefügt. Der Vorlauf ist maximal 5cm lang. Der Hauptkörper hat beispielsweise eine Länge von 5cm bis 12cm. An einer Stelle 38 hat der Bougie einen Außendurchmesser, der im Wesentlichen dem Innendurchmesser 43 abzüglich eventuell dem Innendurchmesser des PEG-Katheters 46 entspricht. Über diese Stelle 38 hinaus kann der Bougie 30 nicht weiter eingeschoben werden, ohne dass ein Widerstand erfolgt. Wird er weiter eingeschoben, wird die PEG-Halteplatte 40 mitgenommen und nach innen, ins Innere gestoßen.

Gegebenenfalls hat der Bougie 30 eine Innenbohrung für den Führungsdraht 18.

Vorzugsweise ist der Bougie 30 aus Kunststoff hergestellt, dabei kann in Co-Extrusion gearbeitet werden.

Figur 11 zeigt eine andere Art eines Bougie 30. Der Bougie 30 ist so ausgelegt, das er einen zylindrischen Hauptkörper 34 hat, dessen Außendurchmesser durch genau den freien Innendurchmesser des PEG-Katheters 46 angepasst ist. Das Ziel ist es, durch den Bougie 30 den PEG-Katheter 46 so auszusteifen, das über den Bougie 30 und den PEG-Katheter 46 gemeinsam eine ausreichend hohe Kraft auf die PEG-Halteplatte 40 ausgeübt werden kann, um diese freistoßen zu können. Hierzu hat der PEG-Bougie 30 eine Länge, die größer ist als die Restlänge des PEG-Katheters 46. Vorzugsweise ist auf dem Hauptkörper 34 ein axial verstellbarer Anschlag 52 vorgesehen, der das untere, freie Ende des PEG-Katheters 46 abstützt. Die Einstellbarkeit ist durch den Doppelpfeil 54 gezeigt.

Der Bougie 30 ist ein Drehteil, eine Längsachse ist in Figur 10 gezeigt. Typische PEG-Halteplatten haben einen Außendurchmesser 41 von 15mm bis 18mm. Der Innendurchmesser 43 liegt typischerweise bei etwa 5mm.

Typischerweise beträgt der Abstand zwischen der Bauchdecke und dem Mageninneren, je nach Patient, etwa 2cm bis 4cm. Man lässt circa 2cm Reihen PEG-Katheter 46 aus der Bauchdecke überstehen. Damit ist der abgeschnittene PEG-Katheter 46 etwa 4cm bis 6cm lang.

Ein Papillotom für die perkutane endoskopische Gastrostomie weist eine vordere Öffnung 11, die sich in einem freien Endbereich eines Katheters 10 und am Austritt eines ersten Lumens 12 befindet, eine hintere Öffnung 9, die weiter entfernt von einem freien Ende 17 ist als die vordere Öffnung 11 und die das erste Lumen 12 mit der Außenseite verbindet, und einen Schneidedraht 16 auf, der sich axial verschiebbar im ersten Lumen 12 befindet, der durch die vordere Öffnung 11 und die hinteren Öffnung 9 verläuft, der sich zwischen der vorderen Öffnung 11 und der hinteren Öffnung 9 auf der Außenseite befindet, wobei bei gespanntem Schneidedraht 16 der freie Endbereich bogenförmig verformt ist und der Schneidedraht 16 zwischen der vorderen Öffnung 11 und der hinteren Öffnung 9 eine quer verlaufende Schnittkante 15 bildet. Die vordere Öffnung 11 ist in einem Abstand von mindestens 3 mm vom freien Ende 17 angeordnet ist. Zwischen dem freien Ende 17 und der vorderen Öffnung 11 ist ein Überstand 20 ausgebildet. Am freien Ende 17 des Katheters 10 ist eine Schneidspitze 20 ausgebildet, die mit dem Schneidedraht 16 verbunden ist und das vorderste Ende des Papillotoms bildet.

## Patentansprüche

1. Papillotom für die perkutane endoskopische Gastrostomie, das aufweist
- einen Handgriff (2)
- einen länglichen, biegbaren Katheter (10), der am Handgriff (2) befestigt ist, der einen freien Endbereich mit einem freien Ende (17) und der mindestens ein erstes Lumen (12) aufweist,
- eine vordere Öffnung (11), die sich im freien Endbereich befindet und das erste Lumen (12) mit einer Außenseite des Katheters (10) verbindet,
- eine hintere Öffnung (9), die sich im freien Endbereich des Katheters (10) befindet, die weiter entfernt vom freien Ende (17) ist als die vordere Öffnung (11) und die das erste Lumen (12) mit der Außenseite verbindet, wobei die vordere Öffnung (11) und die hintere Öffnung (9) in derselben Orientierung zum Katheter (10) angeordnet sind,
- einen Schneidedraht (16), der sich axial verschiebbar im ersten Lumen (12) befindet, der durch die vordere Öffnung (11) und die hinteren Öffnung (9) verläuft, der sich zwischen der vorderen Öffnung (11) und der hinteren Öffnung (9) auf der Außenseite befindet und der im freien Endbereich und im Handgriff (2) festgelegt ist, wobei bei betätigtem Handgriff (2) der Schneidedraht (16) gespannt ist, der freie Endbereich bogenförmig verformt ist und der Schneidedraht (16) zwischen der vorderen Öffnung (11) und der hinteren Öffnung (9) eine quer verlaufende Schnittkante (15) bildet, wobei
die vordere Öffnung (11) in einem Abstand von mindestens 3 mm vom freien Ende (17) angeordnet ist und zwischen dem freien Ende (17) und der vorderen Öffnung (11) ein Überstand (20) ausgebildet ist, **dadurch gekennzeichnet, dass** am freien Ende (17) des Katheters (10) eine Schneidspitze (20) ausgebildet ist, die mit dem Schneidedraht (16) verbunden ist und das vorderste Ende des Papillotoms bildet, dass der Schneidedraht (16) sich zudem zwischen dem freien Ende (17) und der vorderen Öffnung (11) auf der Außenseite befindet, dort die Schneidspitze (20) und die längs verlaufende Schnittkante (19) bildet und dort in derselben Orientierung zum Katheter (10) wie die vordere Öffnung (11) und die hintere Öffnung (9) angeordnet ist,
wobei
a) die Schneidspitze (20) mit einem Metallstück (26) verbunden ist, das sich zumindest über einen Teil des Bereichs zwischen dem freien Ende (17) und der vorderen Öffnung (11) auf der Außenseite befindet, das mit dem Schneidedraht (16) verbunden ist, und das eine längs verlaufende Schnittkante (19) bildet, die in derselben Orientierung zum Katheter (10) wie die vordere Öffnung (11) und die hintere Öffnung (9) angeordnet ist
und/oder
b) eine dritte Öffnung (60) vorgesehen ist, die sich zwischen der vorderen Öffnung (11) und der hinteren Öffnung (9) befindet und wobei der Schneidedraht (16) durch die dritte Öffnung (60) wieder in das erste Lumen (12) eintritt.

2. Papillotom nach Anspruch 1, **dadurch gekennzeichnet, dass** der Schneidedraht (16) die Schneidspitze (20) und/oder das Metallstück ausbildet.

3. Papillotom nach einem der vorangegangenen Ansprüche, **dadurch gekennzeichnet, dass** das freie Ende (17) abgeschrägt ist, wobei eine Spitze der Abschrägung in derselben Orientierung zum Katheter (10) wie die vordere Öffnung (11) und die hintere Öffnung (9) angeordnet ist.

4. Papillotom nach einem der vorangegangenen Ansprüche, **dadurch gekennzeichnet, dass** die vordere Öffnung (11) in einem Abstand von maximal 15 mm vom freien Ende angeordnet ist, insbesondere dass sie in einem Abstand von 5 bis 10 mm und vorzugsweise von 4 bis 7 mm vom freien Ende (17) angeordnet ist.

5. Papillotom nach einem der vorangegangenen Ansprüche, **dadurch gekennzeichnet, dass** der Katheter (10) an einem stationären Teil (3) des Handgriffs (2) befestigt ist, und dass der Schneidedraht (16) an einem bewegbaren Teil (4) des Handgriffs (2) befestigt ist.

6. Papillotom nach Anspruch 1, **dadurch gekennzeichnet, dass** der Handgriff (2) eine Feststellvorrichtung aufweist, und dass die Position des bewegbaren Teil (4) des Handgriffs (2) gegenüber dem stationären Teil (3) des Handgriffs (2) feststellbar ist.

## Claims

1. Papillotome for percutaneous endoscopic gastrostomy, comprising:
- a handle (2);
- an elongate flexible catheter (10) attached to the handle (2), which catheter has a free end region with a free end (17) and has at least one first lumen (12);
- a front opening (11), which is disposed in the free end region and connects the first lumen (12) to an outside of the catheter (10);
- a rear opening (9), which is disposed in the free end region of the catheter (10), is further from the free end (17) than the front opening (11), and connects the first lumen (12) to the outside, the front opening (11) and the rear opening (9) being arranged with the same orientation relative to the catheter (10); and
- a cutting wire (16), which is disposed in an axially displaceable manner in the first lumen (12), extends through the front opening (11) and the rear opening (9), is disposed between the front opening (11) and the rear opening (9) on the outside, and is fixed in the free end region and in the handle (2), wherein, when the handle (2) is actuated, the cutting wire (16) is tensioned, the free end region is deformed in an arc shape, and the cutting wire (16) forms a transversely extending cutting edge (15) between the front opening (11) and the rear opening (9),
the front opening (11) being disposed at a distance of at least 3 mm from the free end (17) and a projection (20) being formed between the free end (17) and the front opening (11), **characterised in that** a cutting tip (20) is provided at the free end (17) of the catheter (10), which cutting tip is connected to the cutting wire (16) and forms the foremost end of the papillotome, and **in that** the cutting wire (16) is additionally disposed between the free end (17) and the front opening (11) on the outside, forms there the cutting tip (20) and the longitudinally extending cutting edge (19), and is arranged there with the same orientation relative to the catheter (10) as the front opening (11) and the rear opening (9),
a) the cutting tip (20) being connected to a metal piece (26), which is disposed at least over a part of the region between the free end (17) and the front opening (11) on the outside, being connected to the cutting wire (16), and forming a longitudinally extending cutting edge (19), which is arranged with the same orientation relative to the catheter (10) as the front opening (11) and the rear opening (9),
and/or
b) a third opening (60) being provided and being disposed between the front opening (11) and the rear opening (9), and
the cutting wire (16) re-entering the first lumen (12) through the third opening (60).

2. Papillotome according to claim 1, **characterised in that** the cutting wire (16) forms the cutting tip (20) and/or the metal piece.

3. Papillotome according to any one of the preceding claims, **characterised in that** the free end (17) is bevelled, a tip of the bevel being disposed with the same orientation relative to the catheter (10) as the front opening (11) and the rear opening (9).

4. Papillotome according to any one of the preceding claims, **characterised in that** the front opening (11) is disposed at a distance of at most 15 mm from the free end, in particular **in that** the front opening is disposed at a distance of from 5 to 10 mm and preferably from 4 to 7 mm from the free end (17).

5. Papillotome according to any one of the preceding claims, **characterised in that** the catheter (10) is attached to a stationary part (3) of the handle (2), and **in that** the cutting wire (16) is attached to a movable part (4) of the handle (2).

6. Papillotome according to claim 1, **characterised in that** the handle (2) has a locking device, and **in that** the position of the movable part (4) of the handle (2) is lockable with respect to the stationary part (3) of the handle (2).

## Revendications

1. Papillotome pour la gastrostomie percutanée endoscopique, présentant
- une poignée (2)
- un cathéter oblong souple (10) qui est fixé sur la poignée (2) qui présente une partie d'extrémité libre avec une extrémité libre (17) et qui présente au moins une première lumière (12),
- une ouverture avant (11) qui se trouve dans la partie d'extrémité libre et relie la première lumière (12) à la face extérieure du cathéter (10),
- une ouverture arrière (9) qui se trouve dans la partie d'extrémité libre du cathéter (10) qui est plus éloignée de l'extrémité libre (17) que l'ouverture avant (11) et qui relie la première lumière (12) à la face extérieure, dans lequel l'ouverture avant (11) et l'ouverture arrière (9) sont disposées dans la même orientation par rapport au cathéter (10),
- un fil de coupe (16) qui se trouve mobile axialement dans la première lumière (12), qui passe à travers l'ouverture avant (11) et l'ouverture arrière (9), qui se trouve entre l'ouverture avant (11) et l'ouverture arrière (9) sur la face arrière et est fixé dans la partie d'extrémité libre et dans la poignée (2), dans lequel lorsque la poignée (2) est actionnée, le fil de coupe (16) est tendu, la partie d'extrémité libre est formée en arc de cercle et le fil de coupe (16) forme une arête de coupe (15) transversale entre l'ouverture avant (11) et l'ouverture arrière (9), dans lequel
l'ouverture avant (11) est disposée dans un espacement d'au moins 3 mm de l'extrémité libre (17) et une saillie (20) est conçue entre l'extrémité libre (17) et l'ouverture avant (11), **caractérisé en ce que** sur l'extrémité libre (17) du cathéter (10), une pointe de coupe (20) est formée qui est reliée au fil de coupe (16) et forme l'extrémité la plus avant du papillotome, **en ce que** le fil de coupe (16) se trouve en outre entre l'extrémité libre (17) et l'ouverture avant (11) sur la face arrière, y forme la pointe de coupe (20) et l'arête de coupe (19) longitudinale et y est disposé dans la même orientation par rapport au cathéter (10) que l'ouverture avant (11) et l'ouverture arrière (9),
dans lequel
a) la pointe de coupe (20) est reliée à une pièce métallique (26) qui se trouve au moins sur une partie de la zone entre l'extrémité libre (17) et l'ouverture avant (11) sur la face extérieure, qui est reliée au fil de coupe (16) et qui forme une arête de coupe (19) longitudinale qui est disposée dans la même orientation par rapport au cathéter (10) que l'ouverture avant (11) et l'ouverture arrière (9) et/ou
b) une troisième ouverture (60) est prévue qui se trouve entre l'ouverture avant (11) et l'ouverture arrière (9) et dans lequel le fil de coupe (16) entre de nouveau dans la première lumière (12) par la troisième ouverture (60).

2. Papillotome selon la revendication 1, **caractérisé en ce que** le fil de coupe (16) forme la pointe de coupe (20) et/ou la pièce métallique.

3. Papillotome selon l'une des revendications précédentes, **caractérisé en ce que** l'extrémité libre (17) est en biseau, dans lequel une pointe du biseau est disposée dans la même orientation par rapport au cathéter (10) que l'ouverture avant (11) et l'ouverture arrière (9).

4. Papillotome selon l'une des revendications précédentes, **caractérisé en ce que** l'ouverture avant (11) est disposée dans un espacement de maximum 15 mm de l'extrémité libre, en particulier **en ce qu'**elle est disposée dans un espacement de 5 à 10 mm et de préférence de 4 à 7 mm de l'extrémité libre (17).

5. Papillotome selon l'une des revendications précédentes, **caractérisé en ce que** le cathéter (10) est fixé sur une partie fixe (3) de la poignée (2) et **en ce que** le fil de coupe (16) est fixé sur une partie mobile (4) de la poignée (2).

6. Papillotome selon la revendication 1, **caractérisé en ce que** la poignée (2) présente un dispositif de réglage et **en ce que** la position de la partie mobile (4) de la poignée (2) peut être réglée par rapport à la partie fixe (3) de la poignée (2).
